# EUROPEAN PATENT APPLICATION

(11) **EP 1 508 601 A1**
(43) Date of publication of application: **23.02.2005**
(21) Application number: 04077310.3
(22) Date of filing: 13.08.2004
(51) Int. Cl.: C09J 4/00, A61L 24/06

(54) **Cyanoacrylate compositions containing anti-microbial agent**

(30) Priority: 21.08.2003 US 644874
(71) Applicant: Closure Medical Corporation, Raleigh, North Carolina 27616 (US)
(72) Inventor: Narang, Upvan, Raleigh, North Carolina 27613 (US)
(74) Representative: Brédeville, Odile Marie

(57) **Abstract**

A monomeric adhesive composition includes a polymerizable monomer, such as a 1,1-disubstituted monomer such as a cyanoacrylate, and an antimicrobial agent, wherein the antimicrobial agent is a phenolic active compound. Examples of suitable phenolic active compounds include halogenated phenol compounds such as chlorinated phenol compounds (such as triclosan) and brominated phenol compounds.

## Description

The invention relates to monomer and polymer adhesive and sealant compositions, which include an anti-microbial agent, and to their production for industrial and medical uses.

Monomer and polymer adhesives are used in both industrial (including household) and medical applications. Included among these adhesives are the 1,1-disubstituted ethylene monomers and polymers, such as the α-cyanoacrylates. Since the discovery of the adhesive properties of such monomers and polymers, they have found wide use due to the speed with which they cure, the strength of the resulting bond formed, and their relative ease of use. These characteristics have made α-cyanoacrylate adhesives the primary choice for numerous applications such as bonding plastics, rubbers, glass, metals, wood, and, more recently, biological tissues.

It is known that monomeric forms of α-cyanoacrylates are extremely reactive, polymerizing rapidly in the presence of even minute amounts of an initiator, including moisture present in the air or on moist surfaces such as animal tissue. Monomers of α-cyanoacrylates are anionically polymerizable or free radical polymerizable, or polymerizable by zwitterions or ion pairs to form polymers. Once polymerization has been initiated, the cure rate can be very rapid.

Medical applications of 1,1-disubstituted ethylene adhesive compositions include use as an alternate or an adjunct to surgical sutures and staples in wound closure as well as for covering and protecting surface wounds such as lacerations, abrasions, bums, stomatitis, sores, and other surface wounds. When an adhesive is applied, it is usually applied in its monomeric form, and the resultant polymerization gives rise to the desired adhesive bond.

For example, polymerizable 1,1-disubstituted ethylene monomers, and adhesive compositions comprising such monomers, are disclosed in U.S. Patent No. 5,328,687 to Leung et al. Suitable methods for applying such compositions to substrates, and particularly in medical applications, are described in, for example, U.S. Patents Nos. 5,582,834, 5,575,997, and 5,624,669, all to Leung et al.

It is known to use cyanoacrylate adhesives to deliver bioactive agents to a wound site. For example, the above patents to Leung et al. disclose such technology in some detail. Examples of such bioactive agents include antimicrobial agents to be released into the wound. U.S. Patents Nos. 5,684,042; 5,753,699; 5,762,919; 5,783,177; 5,811,091; and 5,902,594, all to Greff et al., disclose that an antimicrobially effective amount of an antimicrobial agent may be incorporated into the polymerizable cyanoacrylate ester composition to promote wound healing and retard infection of the wound. See col. 2, lines 50-53 and Abstract of the '699 patent. In order to achieve an antimicrobial effect, an antimicrobial complex of iodine molecules with a biocompatible polymer is used. The iodine/polymer complexes are dispersible in the cyanoacrylate ester. See col. 7, lines 45-48 of the 699 patent. However, the iodine/polymer complexes were not soluble in the cyanoacrylate ester. See Table I, col. 12, line 55 - col. 13, line 14 ofthe 699 patent. See also, U.S. Patents Nos. 5,730,994 and 5,807,563 to Askill et al. and WO 99/18950 to Berger et al.

It is also important to prevent the introduction of microorganisms to the wound site during treatment of the wound. This can generally be accomplished by thorough cleaning of the wound, and by ensuring that sterile products are used to treat the wound. However, even following these precautions, microorganisms can enter the wound either by already being present in surrounding tissues, or by contact with the air during and after treatment.

Cyanoacrylate compositions, at least those for use in medical applications, are generally initially sterile. That is, the compositions as manufactured do not contain live microorganisms. However, through improper handling of the compositions or prolonged or repeated exposure of the compositions to a non-sterile environment, such as with multiple use applicators, microorganisms that are present in the air may be introduced into a cyanoacrylate composition and survive, resulting in the contamination of the composition. Although the source for this characteristic is not understood, it has been observed that cyanoacrylate compositions inherently possess some antimicrobial activity. In particular, cyanoacrylate compositions themselves are believed to prevent the growth of some types of microorganisms within the composition. However, cyanoacrylate compositions by themselves do not possess such a broad spectrum of antimicrobial activity that all amounts of every type of microorganisms would not grow in the compositions.

A way to inactivate microorganisms in the cyanoacrylate compositions is to sterilize the composition. However, sterilization of α-cyanoacrylate adhesive compositions is often difficult to achieve. For example, widely practiced methods of sterilization, such as dry and moist heat sterilization, ionizing radiation, exposure to gas, and aseptic filtration, are not always convenient for use with monomeric cyanoacrylate compositions. Problems sometimes arise due to polymerization of the monomer during the sterilization process. In many cases, sterilization-induced polymerization is so severe that the resulting product is unusable.

Additionally, even if complete sterilization of cyanoacrylate compositions is achieved, such that all microorganisms present in the composition are destroyed, improper handling or exposure to air after sterilization could result in introduction and growth of microorganisms in the cyanoacrylate compositions.

Furthermore, at least in the medical field, there is a real or perceived need or desire for medical products to include active ingredients. For example, such active ingredients can be anti-microbials to prevent the growth of microorganisms at the application site, below and/or above the applied material, or can be anti-inflammatories to provide relief from inflammation and associated problems and discomfort. Preferably, an additive to the composition would provide both anti-microbial and anti-inflammatory properties.

Thus, a need exists for improved cyanoacrylate monomer adhesive compositions, especially for medical uses, that contain anti-microbial and/or anti-inflammatory agents, but where the performance of the adhesive composition is not compromised.

The present invention provides a monomeric adhesive composition comprising an anti-microbial agent, such as particularly a chlorophenol such as triclosan, and a polymerizable monomer, such as a 1,1-disubstituted ethylene monomer.
One aspect of the present invention is a monomeric adhesive composition comprising a polymerizable monomer, and an antimicrobial agent, wherein said antimicrobial agent is a phenolic active compound.
Another aspect of the present invention is a method of making a monomeric adhesive composition, comprising mixing an antimicrobial agent with a polymerizable monomer, wherein said antimicrobial agent is a phenolic active compound.
A further aspect of the present invention is a method of making a sterile, antimicrobial adhesive composition comprising:
placing a mixture of a polymerizable monomer and an antimicrobial agent in a container, wherein said antimicrobial agent is a phenolic active compound,
sealing said container, and
sterilizing the mixture in the container,
wherein said mixture remains stable for at least five minutes after forming the mixture.
A further aspect of the present invention is a method of applying a monomeric adhesive composition, comprising:
applying a monomeric adhesive composition comprising a polymerizable monomer and an antimicrobial agent, wherein said antimicrobial agent is a phenolic active compound and, to a tissue surface; and
allowing the monomeric adhesive composition to polymerize on said tissue surface.
A further aspect of the present invention is a polymer film formed by polymerizing the monomer in the above described composition.
In embodiments, the anti-microbial agent is soluble in the monomer at room temperature and the resultant composition is stable for at least a given amount of time. However, in some specific embodiments, complete solubility may not be required. Production of the composition includes mixing a polymerizable monomer and an anti-microbial agent in a container. The monomeric adhesive composition may be sterilized. Production of the sterilized composition includes placing a polymerizable monomer and an anti-microbial agent in a container, sealing the container and sterilizing the container and the mixture. Optionally, the container used to hold the composition in embodiments of the present invention can be a multi-use container or packaging system. The compositions produced, packaged and sterilized according to the present invention are stable, and have extended utility, as compared to adhesive compositions of the prior art.

According to the present invention, a monomeric adhesive composition comprises an anti-microbial agent and a polymerizable monomer. Generally, the anti-microbial agent is a phenol active compound such as a chlorophenol compound and the polymerizable monomer is a 1,1-disubstituted ethylene monomer, although the present invention is not limited to such materials.

An antimicrobial agent is a compound that either destroys or usefully suppresses the growth or metabolism of a variety of microscopic or submicroscopic life forms, particularly during use of the product containing the antimicrobial agent. Such life forms include, but are not limited to, microorganisms, bacteria, fungi, algae, protozoa, and viruses. The agent preferably has a positive antimicrobial effect against at least microorganisms. Preferably, the agent is effective at reducing the level of microbes in the composition prior to use, and reducing the level of microbes present in a surrounding area during use of the composition, at a commercially acceptable level, or at a level as regulated by an appropriate governing body, such as the U.S. FDA (U.S. Food and Drug Administration), USP (United States Pharmacopia) or the European Pharmacopia, or recommended by associations such as the Cosmetic, Toiletry and Fragrance Association. In this sense, the antimicrobial agent may have the dual function of acting as a preservative in the composition prior to use, in addition to having antimicrobial properties during actual use of the composition.

Preferably, in embodiments of the present invention, the anti-microbial agent is present in the adhesive composition in an amount and form such that the anti-microbial agent can provide its anti-microbial properties subsequent to application of the adhesive composition. Thus, for example, the anti-microbial agent is effective at providing anti-microbial properties to an area under and around a polymerized film of the composition, and/or can leach out of the resultant polymer film to provide active anti-microbial results over an extended period of time.

The present invention, in embodiments, is applicable to single-use containers or applicators, where it is desired to maintain a high degree of prolonged sterility and stability of the composition, by supplementing an optional sterilization treatment by addition of the antimicrobial agent. Likewise, in embodiments, the present invention is also particularly applicable to multiple-use containers or applicators, where it is desired to maintain a high degree of prolonged sterility and stability of the composition against microbial action despite loss of initial sterility upon first use of the composition.

The anti-microbial agent of the present invention operates to destroy microorganisms that may be present or grow in the polymerizable monomer composition prior to use of the composition. The anti-microbial agent of the present invention operates to destroy microorganisms in and around the application site subsequent to polymerization of the composition on the desired application site such as a wound or incision. Although some (and in fact, many) anti-microbial agents may also function as polymerization inhibitors, in embodiments of the present invention the anti-microbial agent does not operate as such, and does not affect polymerization of the monomer material.

In fact, a common problem in the art has been that most anti-microbial agents affect the polymerization rate of the monomer composition, either by acting as initiators or as inhibitors. For example, the antimicrobial complex of iodine molecules with a biocompatible polymer, described above, is generally not soluble or stable in the monomer composition, and tends to cause polymerization of the monomer. Similarly, many known anti-microbial agents such as ammonium salts, bacitracin, and benzalkonium chloride, while effective anti-microbial agents, are in fact initiators of monomer compositions such as cyanoacrylate compositions.

The present invention thus addresses this problem by providing anti-microbial agents that are soluble in the monomer composition, are stable in the monomer composition (i.e., do not cause premature polymerization), and do not affect the polymerization rate of the composition by initiating or inhibiting polymerization. Although some change in the polymerization rate may occur, it is preferred in embodiments that the polymerization rate not be substantially affected or modified. In a preferred embodiment, the anti-microbial agent does not substantially affect the polymerization rate of the monomer. For example, the polymerization rate of the monomer composition with the anti-microbial agent should preferably differ from the polymerization rate of a comparable monomer composition without the anti-microbial agent by no more than about 50%, preferably no more than about 20%.

The compositions of the present invention generally contain an antimicrobial agent, and a polymerizable monomer. The polymerizable monomer, and the composition as a whole, is preferably in liquid form and provides adhesive properties. Other components, known in the art, can also be included in the composition for their known effects and in known amounts.

The antimicrobial agent may be selected from among known antimicrobial agents. Included among said known antimicrobial agents are the various phenolic active compounds, and preferably phenol derivatives, such as halogenated phenol compounds, including chlorinated or brominated phenol compounds. Suitable specific examples include, but are not limited to, tribromophenol, trichlorophenol, tetrachlorophenol, nitrophenol, 3-methyl-4-chloro-phenol, 3,5-dimethyl-4-chlorophenol, phenoxyethanol, dichlorophene, o-phenyl-phenol, m-phenylphenol, p-phenylphenol, 2-benzyl-4-chlorophenol, 2,4-dichloro-3,5-dimethylphenol, 4-chlorothymol, chlorphen, triclosan, fentichlor, phenol, 2-methyl phenol, 3-methyl phenol, 4-methyl phenol, 4-ethyl phenol, 2,4-dimethyl phenol, 2,5-dimethyl phenol, 3,4-dimethyl phenol, 2,6-dimethyl phenol, 4-n-propyl phenol, 4-n-butyl phenol, 4-n-amyl phenol, 4-tert-amyl phenol, 4-n-hexyl phenol, 4-n-heptyl phenol, and mono- and poly-alkyl and aromatic halophenols and their ammonium, alkali metal and alkaline earth metal salts, and mixtures thereof.

In embodiments, the antimicrobial agent is a halogenated phenol, such as a chlorinated phenol or a brominated phenol. In a preferred embodiment, the antimicrobial agent is selected from the group consisting of chlorinated phenol compounds and brominated phenol compounds. Chlorinated phenol compounds that may be used according to the invention include but are not limited to parachlorometaxylenol, triclosan (2,4,4'-trichloro-2 hydroxy di-phenyl ether), p-chlorophenol, 2-chlorophenol, 3-chlorophenol, 4-chlorophenol, 2,4-dichlorophenol, 2,4,6-trichlorophenol, 2,3,4,6-tetrachlorophenol, pentachlorophenol, 4-chlororesorcinol, 4,6-dichlororesorcinol, 2,4,6-trichlororesorcinol, alkylchlorophenols (including p-alkyl-o-chlorophenols, o-alkyl-p-chlorophenols, dialkyl-4-chlorophenol, and tri-alkyl-4-chlorophenol), cyclohexyl p-chlorophenol, o-benzyl p-chlorophenol, o-benxyl-m-methyl p-chlorophenol, o-benzyl-m,m-dimethyl p-chlorophenol, o-phenylethyl p-chlorophenol, o-phenylethyl-m-methyl p-chlorophenol, dichloro-m-xylenol, chlorocresol, o-benzyl-p-chlorophenol, 3,4,6-trichlorphenol, 4-chloro-2-phenylphenol, 6-chloro-2-phenylphenol, o-benzyl-p-chlorophenol, 2,4-dichloro-3,5-diethylphenol, mixtures thereof, and the like. In a preferred embodiment, the antimicrobial agent is a chlorinated phenol compound selected from the group consisting of parachlorometaxylenol, triclosan, p-chlorophenol, 2-chlorophenol, 3-chlorophenol, 4-chlorophenol, 2,4-dichlorophenol, 2,4,6-trichlorophenol, 2,3,4,6-tetrachlorophenol, pentachlorophenol, 4-chlororesorcinol, 4,6-dichlororesorcinol, 2,4,6-trichlororesorcinol, alkylchlorophenols, cyclohexyl p-chlorophenol, o-benzyl p-chlorophenol, o-benxyl-m-methyl p-chlorophenol, o-benzyl-m,m-dimethyl p-chlorophenol, o-phenylethyl p-chlorophenol, o-phenylethyl-m-methyl p-chlorophenol, dichloro-m-xylenol, chlorocresol, o-benzyl-p-chlorophenol, 3,4,6-trichlorphenol, 4-chloro-2-phenylphenol, 6-chloro-2-phenylphenol, o-benzyl-p-chlorophenol, 2,4-dichloro-3,5-diethylphenol, and mixtures thereof.
Specific examples of suitable alkyl chlorophenols include, but are not limited to, methyl p-chlorophenol, ethyl p-chlorophenol, n-propyl p-chlorophenol, n-butyl p-chlorophenol, n-amyl p-chlorophenol, sec-amyl p-chlorophenol, n-hexyl p-chlorophenol, n-heptyl p-chlorophenol, n-octyl p-chlorophenol, o-chlorophenol, methyl o-chlorophenol, ethyl o-chlorophenol, n-propyl o-chlorophenol, n-butyl o-chlorophenol, n-amyl o-chlorophenol, tert-amyl o-chlorophenol, n-hexyl o-chlorophenol, n-heptyl o-chlorophenol, 3-methyl p-chlorophenol, 3,5-dimethyl p-chlorophenol, 6-ethyl-3-methyl p-chlorophenol, 6-n-propyl-3-methyl p-chlorophenol, 6-iso-propyl-3-methyl p-chlorophenol, 2-ethyl-3,5-dimethyl p-chlorophenol, 6-sec-butyl-3-methyl p-chlorophenol, 2-iso-propyl-3,5-dimethyl p-chlorophenol, 6-diethylmethyl-3-methyl p-chlorophenol, 6-iso-propyl-2-ethyl-3-methyl p-chlorophenol, 2-sec-amyl-3,5-dimethyl p-chlorophenol, 2-diethylmethyl-3,5-dimethyl p-chlorophenol, 6-sec-octyl-3-methyl p-chlorophenol, 2,2'-methylene bis (4-chlorophenol), 2,2'-methylene bis (3,4,6-trichlorophenol), mixtures thereof, and the like. Brominated phenol compounds which may be used according to the invention include but are not limited to p-bromophenol, methyl p-bromophenol, ethyl p-bromophenol, n-propyl p-bromophenol, n-butyl p-bromophenol, n-amyl p-bromophenol, sec-amyl p-bromophenol, n-hexyl p-bromophenol, cyclohexyl p-bromophenol, o-bromophenol, tert-amyl o-bromophenol, n-hexyl o-bromophenol, n-propyl-m,m-dimethyl o-bromophenol, 2,2'-methylene bis (4-chloro-6-bromophenol), mixtures thereof, and the like. In a preferred embodiment, the antimicrobial agent is a brominated phenol compound selected from the group consisting of p-bromophenol, methyl p-bromophenol, ethyl p-bromophenol, n-propyl p-bromophenol, n-butyl p-bromophenol, n-amyl p-bromophenol, sec-amyl p-bromophenol, n-hexyl p-bromophenol, cyclohexyl p-bromophenol, o-bromophenol, tert-amyl o-bromophenol, n-hexyl o-bromophenol, n-propyl-m,m-dimethyl o-bromophenol, 2,2'-methylene bis (4-chloro-6-bromophenol), and mixtures thereof.

According to the present invention, it is also envisioned that the anti-microbial agent can possess, and thus provide, additional properties to the adhesive composition. For example, the anti-microbial agent, when properly selected, can also provide anti-inflammatory effects to an area of tissue over which the adhesive composition is applied. Triclosan is one example of such a compound that possesses both anti-microbial and anti-inflammatory properties, and which can beneficially be used in the present invention. In a preferred embodiment, the antimicrobial agent is triclosan.

According to the present invention, the antimicrobial agent is preferably soluble in the monomer composition without significantly adversely affecting the stability of the monomer composition. In addition, in embodiments, the antimicrobial agent in combination with the monomer composition should also be compatible with one or more sterilization procedures. Preferably, the antimicrobial agent is compatible with sterilization processing of said composition.

In embodiments of the present invention, it is preferred that the agent exhibit stability in the monomer composition for at least five minutes after mixing or dissolving the agent in the polymerizable monomer compound, and/or sterilizing a resultant combination. In a preferred embodiment, the antimicrobial agent is soluble in said monomer at room temperature and substantially all of said monomer remains stable for at least five minutes after forming the composition.
More preferably, stability of the monomer composition is maintained for at least one hour, preferably ten hours, and more preferably twenty-four hours after mixing the agent with the polymerizable monomer compound, and/or sterilizing a resultant combination. Preferably, the composition remains stable for at least one hour, more preferably for at least twenty-four hours, after forming the composition. Even more preferably, stability of the monomer composition is maintained for a time period sufficient to provide a commercially significant shelf-life to the monomer composition, or even an extended shelf-life as compared to similar monomer compositions not including such an agent. Preferably, the composition remains stable for at least eighteen months after forming the composition. As used herein, "stability" refers to the resultant composition maintaining a commercially acceptable form for the prescribed amount of time. That is, the composition does not prematurely polymerize or otherwise change form or degrade to the point that the composition is not useful for its intended purpose. Thus, while some polymerization or thickening of the composition may occur, such as can be measured by changes in viscosity of the composition, such change is not so extensive as to destroy or significantly impair the usefulness of the composition.

The amount of antimicrobial agent that is added to the monomer composition depends upon several factors, including, but not limited to, the specific antimicrobial agent being used, the amount of the agent suitable for use in the compositions, and whether and to what extent the agent is regulated by the U.S. FDA (or other appropriate regulatory agencies or bodies of the United States or foreign countries). Typically, the additive is present in an amount of from about 0.001% to about 10%, preferably from about 0.02% to about 2.0%, and more preferably from about 0.1 % to about 1 %, by weight of the total monomer composition.

Preferably, the antimicrobial agent is present in the monomer composition in an amount such that the antimicrobial agent provides the desired antimicrobial effects at the application site. It is believed that once the monomer composition is polymerized, the antimicrobial agent slowly elutes out of the polymer product over time. This slow elution of the anti-microbial agent means that the anti-microbial agent is steadily released from the polymer product, to provide the antimicrobial effect.

It will thus be appreciated that the elution mechanism can be utilized to provide desired antimicrobial effects, such as by tailoring the elution rate and amount of antimicrobial agent available at the application site. For example, the level of antimicrobial effects can be increased by increasing the amount of anti-microbial agent incorporated into the composition. Alternatively, the dissolution or elution rate of the antimicrobial agent from the polymer product can be adjusted by adjusting other factors of the monomer composition, such as other additives, polymer product film strength, biodegradation or absorption rate of the polymer product, and the like.

Preferably, the antimicrobial agent is soluble in a monomer composition at room temperature (i.e., 20-25°C) so that it may be added to the monomer composition without excessive heating of the monomer composition, and so that it remains soluble in the monomer composition during storage of the composition prior to use. The agent is selected such that it is compatible with the monomer (i.e., does not adversely affect polymerization, bond strength, cure properties, or shelf-life).

In embodiments, the adhesive composition has a viscosity of about 1-5000 centipoise, such as 3-600 centipoise, or 5-40 centipoise. The viscosity can be selected according to the proposed use -- e.g., 4-50 centipoises for certain uses and 100-250 centipoises for other uses. Additionally, the composition may be a gel, e.g., 50,000-500,000 centipoise. A gel is a combination of a disperse phase with a continuous phase to produce a semisolid material. The viscosity of the adhesive composition may be measured with a Brookfield Viscometer at 25°C. Additionally, in embodiments where a sterilization treatment is applied, the viscosity of the composition should preferably be maintained or increased by a controlled and acceptable amount after sterilization.

Typically, for medical purposes, an adhesive should have a shelf-life of at least one year; however, an increased shelf-life beyond this provides increased economic advantages to both the manufacturer and the consumer. As used herein, shelf-life refers to the amount of time the container and composition therein can be held at ambient conditions (approximately room temperature) or less, without degradation of the composition and/or container occurring to the extent that the composition and container cannot be used in the manner and for the purpose for which they were intended. Thus, while some degradation to either or both of the composition and container can occur, it must not be to such an extent that the composition and/or container is no longer useable. As used herein, an "extended shelf-life" refers to a shelf-life of at least 12 months, preferably at least 18 months, more preferably at least 24 months, and even more preferably, at least 30 months.

In embodiments, the monomer composition and/or its packaging can be sterilized. In a preferred embodiment, the composition is sterile. Furthermore, whether or not the composition and container is sterilized, the composition can further include one or more suitable preservative, as described below.

Sterilization of the monomer composition and/or its packaging can be accomplished by techniques known to the skilled artisan, and is preferably accomplished by methods including, but not limited to, chemical, physical, and/or irradiation methods. Examples of chemical methods include, but are not limited to, exposure to ethylene oxide or hydrogen peroxide vapor. Examples of physical methods include, but are not limited to, sterilization by heat (dry or moist) or retort canning. Examples of irradiation methods include, but are not limited to, gamma irradiation, electron beam irradiation, and microwave irradiation. Preferably, sterilizing is performed by dry heat, moist heat, gamma irradiation, electron beam irradiation, microwave irradiation, or retort canning. A preferred method is electron beam irradiation, as described in U.S. Patent No. 6,143,805, the entire disclosure of which is incorporated herein by reference. The composition should also show low levels of toxicity to living tissue during its useful life. In preferred embodiments of the present invention, the composition is sterilized to provide a Sterility Assurance Level (SAL) of at least 10⁻³. In embodiments, the Sterility Assurance Level may be at least 10⁻⁴, or may be at least 10⁻⁵, or may be at least 10⁻⁶.

The monomer (including prepolymeric) adhesive composition may include one or more polymerizable monomers. Preferred monomers that may be used in this invention are readily polymerizable, e.g. anionically polymerizable or free radical polymerizable, or polymerizable by zwitterions or ion pairs to form polymers. Such monomers include those that form polymers, that may, but do not need to, biodegrade. Such monomers are disclosed in, for example, U.S. Patents Nos. 5,328,687, 5,928,611 and 6,183,593, U.S. Patent Application Serial No. 09/430,177, filed on October 29, 1999, and U.S. Patent No. 6,183,593, which are hereby incorporated in their entirety by reference herein.

Preferred monomers include 1,1-disubstituted ethylene monomers, such as α-cyanoacrylates including, but not limited to, alkyl α-cyanoacrylates having an alkyl chain length of from about 1 to about 20 carbon atoms or more, preferably from about 3 to about 8 carbon atoms. In a preferred embodiment of the invention, the monomer is a 1,1-disubstituted ethylene monomer. In a more preferred embodiment of the invention, the monomer is a cyanoacrylate.

The α-cyanoacrylates of the present invention can be prepared according to several methods known in the art. U.S. Patents Nos. 2,721,858, 3,254,111, 3,995,641, and 4,364,876, each of which is hereby incorporated in its entirety by reference herein, disclose methods for preparing α-cyanoacrylates.

Preferred α-cyanoacrylate monomers used in this invention include methyl cyanoacrylate, ethyl cyanoacrylate, n-butyl cyanoacrylate, 2-octyl cyanoacrylate, methoxyethyl cyanoacrylate, ethoxyethyl cyanoacrylate, dodecyl cyanoacrylate, 2-ethylhexyl cyanoacrylate, butyl cyanoacrylate, 3-methoxybutyl cyanoacrylate, 2-butoxyethyl cyanoacrylate, 2-isopropoxyethyl cyanoacrylate, 1-methoxy-2-propyl cyanoacrylate, hexyl cyanoacrylate, or dodecylcyanoacrylate.

Other suitable cyanoacrylates for use in the present invention also include, but are not limited to, alkyl ester cyanoacrylate monomers such as those having the formula 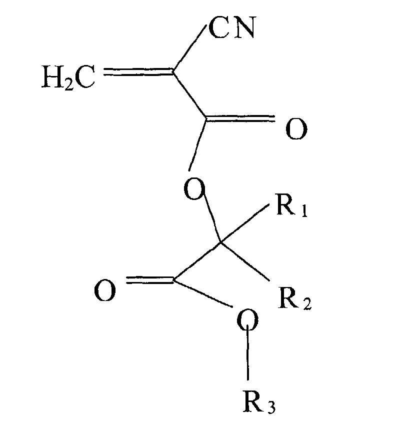 wherein R₁ and R₂ are, independently H, a straight, branched or cyclic alkyl, or are combined together in a cyclic alkyl group, and R₃ is a straight, branched or cyclic alkyl group. Preferably, R₁ is H or a C₁, C₂ or C₃ alkyl group, such as methyl or ethyl; R₂ is H or a C₁, C₂ or C₃ alkyl group, such as methyl or ethyl; and R₃ is a C₁ -C₁₆ alkyl group, more preferably a C₁ -C₁₀ alkyl group, such as methyl, ethyl, propyl, butyl, pentyl, hexyl, heptyl, octyl, nonyl or decyl, and even more preferably a C₂, C₃ or C₄ alkyl group. Such alkyl ester cyanoacrylates and other suitable monomers are disclosed in, for example, U.S. Patent Applications Nos. 09/630,437, filed August 2, 2000, and 09/919,877, filed August 2, 2001, the entire disclosures of which are incorporated herein by reference.

Examples of preferred alkyl ester cyanoacrylates include, but are not limited to, butyl lactoyl cyanoacrylate (BLCA), butyl glycoloyl cyanoacrylate (BGCA), ethyl lactoyl cyanoacrylate (ELCA), and ethyl glycoloyl cyanoacrylate (EGCA). BLCA may be represented by the above formula, wherein R₁ is H, R₂ is methyl and R₃ is butyl. BGCA may be represented by the above formula, wherein R₁ is H, R₂ is H and R₃ is butyl. ELCA may be represented by the above formula, wherein R₁ is H, R₂ is methyl and R₃ is ethyl. EGCA may be represented by the above formula, wherein R₁ is H, R₂ is H and R₃ is ethyl.

The composition may optionally also include at least one other plasticizing agent that assists in imparting flexibility to the polymer formed from the monomer. The plasticizing agent preferably contains little or no moisture and should not significantly affect the stability or polymerization of the monomer. Examples of suitable plasticizers include but are not limited to tributyl citrate, acetyl tri-n-butyl citrate (ATBC), polymethylmethacrylate, silicone oils, siloxanes, and others as listed in U.S. Patent No. 6,183,593, the disclosure of which is incorporated in its entirety by reference herein. Specific examples of the silicone oils and siloxanes include, for example, but are not limited to, polydimethylsiloxane, hexadimethylsilazane.

The composition may also optionally include at least one thixotropic agent. Suitable thixotropic agents are known to the skilled artisan and include, but are not limited to, silica gels such as those treated with a silyl isocyanate, and optionally surface treated titanium dioxide. Examples of suitable thixotropic agents and thickeners are disclosed in, for example, U.S. Patent No. 4,720,513, and U.S. Patent No. 6,310,166, the disclosures of which are hereby incorporated in their entireties by reference herein.

The composition may optionally also include thickeners. Suitable thickeners may include poly (2-ethylhexyl methacrylate), poly(2-ethylhexyl acrylate) and others as listed in U.S. Patent No. 6,183,593, the disclosure of which is incorporated by reference herein in its entirety.

The composition may also optionally include at least one natural or synthetic rubber to impart impact resistance. Suitable rubbers are known to the skilled artisan. Such rubbers include, but are not limited to, dienes, styrenes, acrylonitriles, and mixtures thereof. Examples of suitable rubbers are disclosed in, for example, U.S. Patents Nos. 4,313,865 and 4,560,723, the disclosures of which are hereby incorporated in their entireties by reference herein.

The composition may optionally also include one or more stabilizers, preferably both at least one anionic vapor phase stabilizer and at least one anionic liquid phase stabilizer. These stabilizing agents may inhibit premature polymerization. Suitable stabilizers may include those listed in U.S. Patent No. 6,183,593, the disclosure of which is incorporated by reference herein in its entirety. Furthermore, certain stabilizers may also function as active agents, such as, for example, various acidic anti-microbials, as identified above.

The stability, and thus the shelf-life, of some monomeric adhesive compositions can be further enhanced and extended through careful regulation of the packaging. Treated (e.g., fluorinated polymer) packaging such as that disclosed in copending U.S. Patent Application Serial No. 09/430,289, filed October 29, 1999, which is hereby incorporated by reference herein in its entirety, is preferred and may reduce the amount of stabilizer that is combined into the composition. As mentioned above, certain stabilizers including, but not limited to, certain acidics can also function as active agents. In this case, the amount of the active agent/stabilizer material is either not reduced below a level to provide the desired effect, or a further active agent/non-stabilizing agent is added to ensure that the desired effect is provided.

The compositions may also include pH modifiers to control the rate of degradation of the resulting polymer, as disclosed in U.S. Patent No. 6,143,352, the entire disclosure of which is hereby incorporated by reference herein in its entirety.

Compositions of the present invention may also include at least one biocompatible agent effective to reduce active formaldehyde concentration levels produced during *in vivo* biodegradation of the polymer (also referred to herein as "formaldehyde concentration reducing agents"). Preferably, this component is a formaldehyde scavenger compound. Examples of formaldehyde scavenger compounds useful in this invention include sulfites; bisulfites; mixtures of sulfites and bisulfites, etc. Additional examples of formaldehyde scavenger compounds useful in this invention and methods for their implementation can be found in U.S. Patents Nos. 5,328,687, 5,514,371, 5,514,372, 5,575,997, 5,582,834 and 5,624,669, all to Leung et al., which are hereby incorporated herein by reference in their entireties.

To improve the cohesive strength of adhesives formed from the compositions of this invention, difunctional monomeric cross-linking agents may be added to the monomer compositions of this invention. Such crosslinking agents are known. U.S. Patent No. 3,940,362 to Overhults, which is hereby incorporated herein in its entirety by reference, discloses exemplary cross-linking agents.

The compositions of this invention may further contain fibrous reinforcement and colorants such as dyes, pigments, and pigment dyes. Examples of suitable fibrous reinforcement include PGA microfibrils, collagen microfibrils, and others as described in U.S. Patent No. 6,183,593, the disclosure of which is incorporated by reference herein in its entirety.

In embodiments of the present invention, the composition and/or its applicator may contain materials such as a polymerization initiator, accelerator, rate-modifier, and/or cross-linking agent for initiating polymerization and/or cross-linking of the polymerizable monomer material. Suitable materials and applicators and packaging systems are disclosed in U.S. Patents Nos. 5,928,611, 6,352,704 and 6,455,064 and U.S. Patent Applications Serial Nos. 09/430,177, 09/430,289, 09/430,290, and 09/430,180 filed October 29, 1999; 09/385,030 filed August 30, 1999; and 09/176,889 filed October 22, 1998; the entire disclosures of which are incorporated herein by reference.

According to the present invention, any suitable applicator can be used to apply the composition to the affected areas of skin. Suitable applicators and packaging systems are disclosed in, for example, U.S. Patents Nos. 5,928,611, 6,352,704 and 6,455,064 and U.S. Patent Applications Serial Nos. 09/430,177, 09/430,289, 09/430,290, and 09/430,180 filed October 29, 1999; 09/385,030 filed August 30, 1999; 09/176,889 filed October 22, 1998, and 09/898,006 filed July 5, 2001; the entire disclosures of which are incorporated herein by reference.

### EXAMPLES

### Example 1:

A 2-octyl cyanoacrylate monomer composition is prepared by adding 1 wt% triclosan to 2 mL of 2-octyl cyanoacrylate monomer. The mixture is sealed in a glass vial or plastic vial and stirred.

The characteristics of thc composition are observed at about one minute after preparation and later at least twenty-four hours after preparation. The results of the observations show that the solution remains clear, indicating that triclosan is soluble in the monomer and does not cause premature polymerization.

### Example 2:

A 2-octyl cyanoacrylate monomer composition is prepared as in Example 1 by adding 1 wt% triclosan to 2 mL of 2-octyl cyanoacrylate monomer. The mixture is sealed in a glass vial or plastic vial and stirred.

After sitting for about twenty-four hours, the mixture contained in the plastic vial is sterilized using electron beam sterilization to a Sterility Assurance Level (SAL) of 10⁻⁶. After sitting for about twenty-four hours, the mixture contained in the glass vial is sterilized using dry heat sterilization to a Sterility Assurance Level (SAL) of 10-6. The characteristics of the sterilized compositions are observed at about one minute after sterilization and later at least twenty-four hours after sterilization. The results of the observations show that the solutions remains clear, indicating that triclosan is compatible with electron beam sterilization and dry heat sterilization processing and does not cause premature polymerization during or after sterilization.

While the invention has been described with reference to preferred embodiments, the invention is not limited to the specific examples given, and other embodiments and modifications can be made by those skilled in the art without departing from the spirit and scope of the invention.

## Claims

1. A monomeric adhesive composition comprising:
a polymerizable monomer, and
an antimicrobial agent,
wherein said antimicrobial agent is a phenolic active compound.

2. The composition of claim 1, wherein said antimicrobial agent is a halogenated phenol compound.

3. The composition of claim 1, wherein said antimicrobial agent is selected from the group consisting of chlorinated phenol compounds and brominated phenol compounds.

4. The composition of claim 1, wherein said antimicrobial agent is a chlorinated phenol compound selected from the group consisting of parachlorometaxylenol, triclosan, p-chlorophenol, 2-chlorophenol, 3-chlorophenol, 4-chlorophenol, 2,4-dichlorophenol, 2,4,6-trichlorophenol, 2,3,4,6-tetrachlorophenol, pentachlorophenol, 4-chlororesorcinol, 4,6-dichlororesorcinol, 2,4,6-trichlororesorcinol, alkylchlorophenols, cyclohexyl p-chlorophenol, o-benzyl p-chlorophenol, o-benxyl-m-methyl p-chlorophenol, o-benzyl-m,m-dimethyl p-chlorophenol, o-phenylethyl p-chlorophenol, o-phenylethyl-m-methyl p-chlorophenol, dichloro-m-xylenol, chlorocresol, o-benzyl-p-chlorophenol, 3,4,6-trichlorphenol, 4-chloro-2-phenylphenol, 6-chloro-2-phenylphenol, o-benzyl-p-chlorophenol, 2,4-dichloro-3,5-diethylphenol, and mixtures thereof.

5. The composition of claim 1, wherein said antimicrobial agent is triclosan.

6. The composition of claim 1, wherein said antimicrobial agent is a brominated phenol compound selected from the group consisting of p-bromophenol, methyl p-bromophenol, ethyl p-bromophenol, n-propyl p-bromophenol, n-butyl p-bromophenol, n-amyl p-bromophenol, sec-amyl p-bromophenol, n-hexyl p-bromophenol, cyclohexyl p-bromophenol, o-bromophenol, tert-amyl o-bromophenol, n-hexyl o-bromophenol, n-propyl-m,m-dimethyl o-bromophenol, 2,2'-methylene bis (4-chloro-6-bromophenol), and mixtures thereof.

7. The composition of claim 1, wherein said antimicrobial agent is soluble in said monomer at room temperature and substantially all of said monomer remains stable for at least five minutes after forming the composition.

8. The composition of claim 1, wherein said antimicrobial agent is compatible with sterilization processing of said composition.

9. The composition of claim 1, wherein said composition remains stable for at least one hour after forming the composition.

10. The composition of claim 1, wherein said composition remains stable for at least twenty-four hours after forming the composition.

11. The composition of claim 1, wherein said composition remains stable for at least eighteen months after forming the composition.

12. The composition of claim 1, wherein said anti-microbial agent does not substantially affect the polymerization rate of the monomer.

13. The composition of claim 1, wherein said monomer is a 1,1-disubstituted ethylene monomer.

14. The composition of claim 1, wherein said monomer is a cyanoacrylate.

15. The composition of claim 1, wherein said composition is sterile.

16. A method of making a monomeric adhesive composition, comprising mixing an antimicrobial agent with a polymerizable monomer, wherein said antimicrobial agent is a phenolic active compound.

17. The method of claim 16, wherein said antimicrobial agent is a halogenated phenol compound.

18. The method of claim 16, wherein said antimicrobial agent is selected from the group consisting of chlorinated phenol compounds and brominated phenol compounds.

19. The method of claim 16, wherein said antimicrobial agent is a chlorinated phenol compound selected from the group consisting of parachlorometaxylenol, triclosan, p-chlorophenol, 2-chlorophenol, 3-chlorophenol, 4-chlorophenol, 2,4-dichlorophenol, 2,4,6-trichlorophenol, 2,3,4,6-tetrachlorophenol, pentachlorophenol, 4-chlororesorcinol, 4,6-dichlororesorcinol, 2,4,6-trichlororesorcinol, alkylchlorophenols, cyclohexyl p-chlorophenol, o-benzyl p-chlorophenol, o-benxyl-m-methyl p-chlorophenol, o-benzyl-m,m-dimethyl p-chlorophenol, o-phenylethyl p-chlorophenol, o-phenylethyl-m-methyl p-chlorophenol, dichloro-m-xylenol, chlorocresol, o-benzyl-p-chlorophenol, 3,4,6-trichlorphenol, 4-chloro-2-phenylphenol, 6-chloro-2-phenylphenol, o-benzyl-p-chlorophenol, 2,4-dichloro-3,5-diethylphenol, and mixtures thereof.

20. The method of claim 16, wherein said antimicrobial agent is triclosan.

21. The method of claim 16, wherein said antimicrobial agent is soluble in said monomer at room temperature and substantially all of said monomer remains stable for at least five minutes after forming the composition.

22. The method of claim 16, wherein said antimicrobial agent is compatible with sterilization processing of said composition.

23. The method of claim 16, wherein said monomer is a 1,1-disubstituted ethylene monomer.

24. The method of claim 16, wherein said monomer is a cyanoacrylate.

25. The method of claim 16, wherein said composition is sterile.

26. A method of making a sterile, antimicrobial adhesive composition comprising:
placing a mixture of a polymerizable monomer and an antimicrobial agent in a container, wherein said antimicrobial agent is a phenolic active compound,
sealing said container, and
sterilizing the mixture in the container,
wherein said mixture remains stable for at least five minutes after forming the mixture.

27. The method of claim 26, wherein said sterilizing is performed by dry heat, moist heat, gamma irradiation, electron beam irradiation, microwave irradiation, or retort canning.

28. A method of applying a monomeric adhesive composition, comprising:
applying a monomeric adhesive composition comprising a polymerizable monomer and an antimicrobial agent, wherein said antimicrobial agent is a phenolic active compound and, to a tissue surface; and
allowing the monomeric adhesive composition to polymerize on said tissue surface.

29. A polymer film formed by polymerizing the monomer in the composition of claim 1.
